(19) Europäisches Patentamt
European Patent Office
Office européen des brevets

(11) **EP 4 345 834 A1**

(12) **EUROPEAN PATENT APPLICATION**

(43) Date of publication:
**03.04.2024 Bulletin 2024/14**

(21) Application number: **22198722.5**

(22) Date of filing: **29.09.2022**

(51) International Patent Classification (IPC):
**G16H 30/20** (2018.01)   **G16H 50/20** (2018.01)

(52) Cooperative Patent Classification (CPC):
**G16H 50/20; G16H 30/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **PEZZOTTI, Nicola**
  Eindhoven (NL)
• **SCHNELLBÄCHER, Nikolas David**
  Eindhoven (NL)
• **DE WEERDT, Elwin**
  5656AG Eindhoven (NL)

(74) Representative: **Philips Intellectual Property &
Standards
High Tech Campus 52
5656 AG Eindhoven (NL)**

(54) **STORAGE OF MEDICAL IMAGE DATA USING NEURAL NETWORKS**

(57)     Disclosed herein is a medical system (100, 300, 500) comprising a memory (110) storing machine executable instructions (112). The medical system further comprises a computational system (104), wherein execution of the machine executable instructions causes the computational system to: receive (200) medical image data (114) descriptive of a field of view (309) of a subject (318); receive (202) an initialized neural network (116), wherein the initialized neural network is configured to output an image value in response to receiving a set of continuously variable coordinates spanning the field of view of the subject; provide (204) a trained neural network (118) by training the initialized neural network to reproduce the medical image data; and store (208) the trained neural network in a persistent storage medium (110, 110').

Fig. 1

EP 4 345 834 A1

## Description

FIELD OF THE INVENTION

The invention relates to medical imaging, in particular to the storage of medical image data.

BACKGROUND OF THE INVENTION

Various medical imaging techniques such as Magnetic Resonance Imaging (MRI), Computed Tomography, Positron Emission Tomography, and Single Photon Emission Tomography enable detailed visualization of anatomical structure of a subject.

The arXive article Mueller et. al. "Instant Neural Graphics Primitives with a Multiresolution Hash Encoding," arXiv:2201.05989 (later published as ACM Trans. Graph, 41(2022), Article 102) discloses that neural graphics primitives, parameterized by fully connected neural networks, can be costly to train and evaluate. It reduces costs with an input encoding that permits the use of a smaller network without sacrificing quality, thus significantly reducing the number of floating point and memory access operations: a small neural network is augmented by a multiresolution hash table of trainable feature vectors whose values are optimized through stochastic gradient descent. The multiresolution structure allows the network to disambiguate hash collisions, making for a simple architecture that is trivial to parallelize on modern GPUs.

SUMMARY OF THE INVENTION

The invention provides for a medical system, a data structure, a method, and a computer program in the independent claims. Embodiments are given in the dependent claims.

A common issue in medical imaging is that many images during an examination may be generated. Aggregated over many subjects, there may be a need to store an immense number of images for later use. Embodiments may provide for a means of storing medical image data by training a neural network to reproduce the medical image data. This may have several advantages. One advantage is that the image may be accurately compressed using the neural network. Another advantage is that the resolution may be adjusted arbitrarily in a way that does not produce confusing image artifacts.

In one aspect the invention provides for a medical system that comprises a memory storing machine-executable instructions. The medical system further comprises a computational system. Execution of the machine-executable instructions causes the computational system to receive medical image data descriptive of a field of view of a subject. The medical image data as used herein encompasses images acquired using a medical imaging system, such as a tomographic medical imaging system that are in image space.

Execution of the machine-executable instructions further causes the computational system to receive an initialized neural network. The initialized neural network is configured to output an image value in response to receiving a set of continuously variable coordinates spanning the field of view of the subject. That is to say the initialized neural network receives a set of coordinates which are continuously variable and in response the initialized neural network outputs an image value. The set of continuously variable coordinates may take different forms in different examples. In one case the medical image data could be a two-dimensional image in which case there would be two continuously variable coordinates. If the image data is three-dimensional image data such as three-dimensional computed tomography or magnetic resonance imaging data or it is two-dimensional and time dependent then there would be three continuously variable coordinates. There may be up to three spatial coordinates, there may also be a time dependency. In some other examples there may be multiple images encoded in the medical image data. For example, it may be a stack of slices. In other examples there may be multiple images using different image contrasts or acquisition or even different image modalities that are grouped together. A coordinate may be used to select which image is desired to be viewed or reproduced.

Execution of the machine-executable instructions further causes the computational system to provide a trained neural network by training the initialized neural network to reproduce the medical image data. The trained neural network is configured to receive the set of continuously variable coordinates and output an image value. The initialized neural network may be trained by repeatedly taking a value of a pixel or voxel of the medical image data and using this as a ground truth data while inputting the coordinates. The trained neural network can be provided by training the initialized neural network until it accurately reproduces the medical image data. Different means may be used, for example it may be done using a typical back propagation or it may even be done using a genetic algorithm learning technique. Different types of neural networks may be used. In one example the trained neural network is a CPPN neural network. In other examples, the trained neural network or the initialized neural network may be implemented using multiple fully connected layers.

In another embodiment execution of the machine-executable instructions further causes the computational system to store the trained neural network in a persistent storage medium. The trained neural network may take different forms in different examples. In one case the trained neural network may have a standard or predetermined network architecture. In this case storing the trained neural network may encompass just storing the various weights for the trained neural network. In other examples, the exact architecture or structure of the trained neural network may be open in which case storing the trained neural network may be a combination of storing metadata which is descriptive of the structure of the trained neural network as well as storing the weighting factors.

This embodiment may be beneficial because it may provide a means of reducing the storage size of the medical image data. Another potential advantage is that since the trained neural network outputs the image value in response to receiving the set of continuously variable coordinates, the resolution of any image reconstructed using the trained neural network may have an arbitrary resolution. The reproduction using a neural network may result in a slightly blurred image when used to generate an image with a higher resolution than what the neural network was trained for. This may have advantages in that this method of super resolution does not add artifacts to an image generated using the trained neural network. This means that the resolution of the resulting image can be chosen freely and it is constructed in a way that does not produce misleading artifacts to mislead a technician or a physician.

In another embodiment execution of the machine-executable instructions further causes the computational system to delete the medical image data after providing the trained neural network. As the medical image data is deleted the trained neural network becomes a means of storing or reproducing the medical image data. This may provide for a more compact means of storing the medical image data. In addition, portions of the medical image data are available without the need to decompress the medical image data. This may therefore be a superior and more convenient means of archiving medical image data.

In another embodiment the medical image data comprises multiple image types of the field of view. The set of continuously variable coordinates comprises an image selection coordinate used to select one of the multiple image types. For example, the multiple image types may be images acquired using different magnetic resonance imaging protocols, this change in for example different types of image weighting in magnetic resonance imaging is typically referred to as contrast. In other examples the multiple image types may also be useful for storing multi-energy computed tomography data. For example, in dual-energy spectral CT two energy images may be stored together and used for fully spectral systems with an arbitrary number of energy bins. There may be an image stored in the neural network for each of these energy bins. This embodiment may be advantageous because the images are all of the subject. As they are related to each other it may be possible to jointly compress multiple images together and achieve a higher compression or storage benefit.

In another embodiment the trained neural network comprises multiple fully connected layers. For example, there may be an input layer, several intermediate layers which may provide for conditioning of the input, and then the multiple fully connected layers. After the fully connected layers there may be an output layer and there may also be some optional layers between the fully connected layers and the output layer. The use of the multiple fully connected layers may be beneficial because this may provide for an efficient means of encoding the medical image data as a trained neural network. The number of fully connected layers may be selected when the neural network is initialized. The compression of the medical image data by the trained neural network enables compression to about 30% of the original size. The number of fully connected layers can be selected during the initialization of the initialized neural network to choose the appropriate number.

In another embodiment the trained neural network comprises three fully connected layers.

In another embodiment the trained neural network comprises four fully connected layers.

In another embodiment the trained neural network comprises five fully connected layers.

In another embodiment the trained neural network comprises six fully connected layers.

In another embodiment the multiple fully connected layers each have fewer than 10,000 nodes. Limiting the size of the nodes in the fully connected layers may have the benefit of limiting the size of the trained neural network and thereby providing compression over the medical image data.

In another embodiment the multiple fully connected layers each have below 5,000 nodes.

In another embodiment each of the fully connected layers have below 3,000 nodes.

In another embodiment the multiple fully connected layers each have below 2,000 nodes.

In another embodiment each of the multiple fully connected layers has below 1,000 nodes.

In another embodiment the initialized neural network is trained with the medical imaging data using a back propagation training algorithm. This embodiment may be beneficial because it may provide a means for training the trained neural network such that it accurately reproduces the medical image data.

In another embodiment the initialized neural network is trained using a genetic algorithm optimization technique. The use of a genetic algorithm optimization technique may be beneficial in some situations where the gradient descent in a back propagation training algorithm does not accurately train the neural network.

In another embodiment the trained neural network has a memory storage size less than a memory storage size of the medical image data. This embodiment may be beneficial because it may provide for a means of compressing the storage of the medical image data.

In another embodiment the trained neural network is stored in the persistent storage medium by placing it into a data structure. This embodiment may be beneficial because it may provide for a means of storing the medical image data within a context. For example, the data storage may have additional metadata or descriptors descriptive of the medical image data.

In another embodiment the data structure is a digital imaging and communications in medicine file or DICOM file. This embodiment may be beneficial because it may provide for a standardized means of storing the medical image data as a trained neural network.

In another embodiment the medical image data and the image value are preferably complex valued. This embodiment may be beneficial because it may provide for an accurate means of storing large quantities of complex medical image data in a compact form. When the medical image data and the image value are complex valued, then the image value will have terms that are used to represent a complex value.

In another embodiment the medical system further comprises a medical imaging system that is configured to acquire measurement data descriptive of the field of view of the subject. For example, the medical imaging system may be a tomographic medical imaging system such as a magnetic resonance imaging system or a computed tomography system. Execution of the machine-executable instructions further causes the computational system to control the medical imaging system to acquire the measurement data. In the case of a magnetic resonance imaging system, the magnetic resonance imaging system would be controlled with pulse sequence commands and be used to acquire k-space data. In the case of a computed tomography system the measurement data will be X-ray profile data.

Execution of the machine-executable instructions further causes the computational system to reconstruct the medical image data from the measurement data. Once the medical image data has been reconstructed then it may be used to provide the training data for the initialized neural network that is used for obtaining the trained neural network for that image reconstruction, by training said neural network with said training data.

The medical system may take different forms in different examples. In the case where the medical system is only the computational system the medical system can be implemented or integrated into a variety of systems. For example, it could be part of a cloud-based system where the computational system is provided on demand. In other examples, the computational system may be integrated into a workstation of a radiology department. In other examples, the computational system could be integrated into the control of a magnetic resonance imaging system.

In another embodiment the medical imaging system is a tomographic medical imaging system.

In another embodiment the medical imaging system is a magnetic resonance imaging system.

In another embodiment the medical imaging system is a positron emission tomography system.

In another embodiment the medical imaging system is a single photon emission tomography system.

In another embodiment the medical imaging system is an X-ray system. For example, it may be a digital X-ray system.

In another embodiment the medical imaging system is a fluoroscope system. For example, it may be a digital fluoroscope.

In another embodiment the medical imaging system is a computed tomography system.

In another embodiment the medical imaging system is an ultrasound imaging system.

In another embodiment the medical imaging system is an intravascular ultrasound system.

In another embodiment the medical imaging system is an optical coherence tomography system.

In another embodiment execution of the machine-executable instructions further causes the computational system to form a medical image by repeatedly obtaining the medical image value by inputting a predetermined set of discrete spatial coordinates into the trained neural network. The predetermined set of discrete spatial coordinates spans at least a portion of the medical image. This embodiment may be beneficial because it may provide for a means of accurately recreating or rendering an image from the medical image data.

The medical image may be formed in different ways depending upon the data that is encoded in the trained neural network. If the data is two-dimensional then the predetermined set of discrete spatial coordinates may be used to select a portion of the two-dimensional medical image data or even to change the resolution, for example to increase or decrease it. An advantage of using the neural network is that it provides an accurate interpolation type or estimate of the medical image data in between the discrete values that were used to train it. Likewise, if the medical image data is three-dimensional the predetermined set of discrete spatial coordinates can be used to construct three-dimensional datasets or two-dimensional datasets. Both the two and three-dimensional datasets may also be time-dependent and by selecting particular time coordinates in the predetermined set of discrete spatial coordinates the particular time when the images are rendered may also be selected.

In another embodiment the predetermined set of discrete spatial coordinates preferably has a spatial resolution that is higher than the medical image data.

In another embodiment the predetermined set of discrete spatial coordinates preferably has a spatial resolution that is lower than the medical image data.

In another embodiment the medical image data comprises three-dimensional image data. The predetermined set of discrete spatial coordinates forms a two-dimensional surface. This two-dimensional surface may for example be a manifold. This may be used to select two-dimensional planes for which image data is constructed that do not lie flat within a section or plane of the three-dimensional image data. This may for example allow the formation of images that form coordinate systems based on the anatomical structures of a subject. In other cases, it may enable the construction of images around the coordinates system of the measurement system. For example, for an intravascular ultrasound system a two-dimensional surface could be defined as a cylinder around the measurement data acquired by the intravascular ultrasound system. To perform these renderings, one only needs to know a translation between the coordinates system of the two-dimensional surface and the set of continuously variable coordinates that span the field of view of the subject. It enables very complex viewing planes to be formed very simply.

In another embodiment the medical image data is time dependent. The set of continuously variable coordinates comprises a time coordinate. This embodiment may be beneficial because it may enable the interpolation of the medical image data at arbitrary times.

In another aspect the invention provides for a data structure for storing at least one image. The data structure comprises an image identifier and a set of weights for a trained neural network. In some examples the data structure also comprises metadata which is descriptive of a topology of the trained neural network. The trained neural network is configured to output an image value in response to receiving a set of continuously variable coordinates spanning the at least one image. The image is readable by repeatedly inputting the set of continuously variable coordinates. This embodiment may be beneficial because it may provide for an extremely compact and accurate means of storing at least one image.

In another embodiment the data structure is a digital imaging and communications in medicine file.

In another aspect the invention provides for a medical imaging method. The method comprises receiving medical image data descriptive of a field of view of a subject. This medical image data may be in image space. The method further comprises receiving an initialized neural network. The initialized neural network is configured to output an image value in response to receiving a set of continuously variable coordinates spanning the field of view of the subject. The method further comprises providing a trained neural network by training the initialized neural network to reproduce the medical image data. The method further comprises storing the trained neural network in a persistent storage medium.

In another aspect the invention provides for a computer program that comprises machine-executable instructions for execution by a computational system. The computer program may for example store the machine-executable instructions on a non-transitory storage medium. Execution of the machine-executable instructions causes the computational system to receive medical image data descriptive of a field of view of a subject. Execution of the machine-executable instructions further causes the computational system to receive an initialized neural network. The initialized neural network is configured to output an image value in response to receiving a set of continuously variable coordinates spanning the field of view of the subject. Execution of the machine-executable instructions further causes the computational system to provide a trained neural network by training the initialized neural network to reproduce the medical image data. Execution of the machine-executable instructions further causes the computational system to store the trained neural network in a persistent storage medium.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multicore processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Measurement data is defined herein as being recorded measurements made by a medical imaging system or a tomographic medical imaging system descriptive of a subject. The measurement data may be reconstructed into medical image data. A medical image data is defined herein as being the reconstructed two- or three-dimensional, and possibly temporal, visualization of anatomic data contained within the medical imaging data. This visualization can be performed using a computer.

K-space data is defined herein as being the recorded measurements of radio frequency signals emitted by atomic spins using the antenna of a magnetic resonance apparatus during a magnetic resonance imaging scan. Magnetic resonance data is an example of measurement data.

A Magnetic Resonance Imaging (MRI) image or MR image is defined herein as being the reconstructed two- or three-dimensional visualization of anatomic data contained within the magnetic resonance imaging data. This visualization can be performed using a computer.

BRIEF DESCRIPTION OF THE DRAWINGS

In the following preferred embodiments of the invention will be described, by way of example only, and with reference to the drawings in which:

Fig. 1 illustrates an example of a medical system;
Fig. 2 shows a flow chart which illustrates a method of using the medical system of Fig. 1;
Fig. 3 illustrates a further example of a medical system;
Fig. 4 shows a flow chart which illustrates a method of using the medical system of Fig. 1;
Fig. 5 illustrates a further example of a medical system;
Fig. 6 illustrates a neural network architecture;
Fig. 7 illustrates an example of a data structure;
Fig. 8 shows a flowchart which illustrates a compression scheme using the trained neural network;
Fig. 9 shows an image compressed with a neural network;
Fig. 10 shows an image using strong jpeg compression;
Fig. 11 compares a jpeg compressed image, the original image, and a neural network compressed image; and
Fig. 12 illustrates image interpolation using a trained neural network.

DETAILED DESCRIPTION OF EMBODIMENTS

Like numbered elements in these figures are either equivalent elements or perform the same function. Elements which have been discussed previously will not necessarily be discussed in later figures if the function is equivalent.

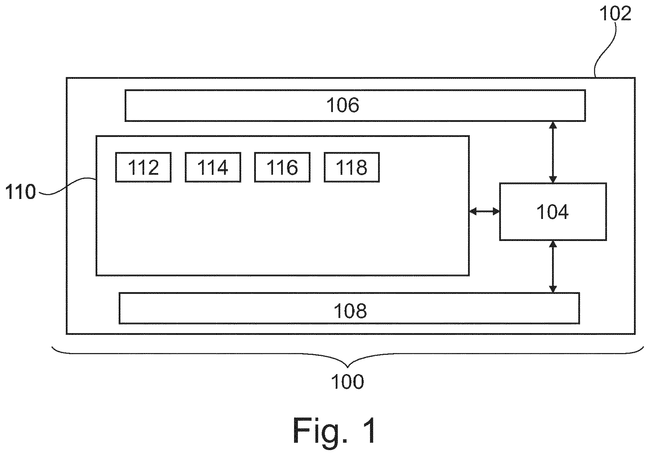

Fig. 1 illustrates an example of a medical system 100. The medical system 100 is shown as comprising a computer 102 that comprises a computational system 104. The computer 102 is intended to represent one or more computers or computing systems or environments that have been located at one or more locations. Likewise, the computational system 104 is intended to represent one or more computational systems or computing cores that may also be located at one or more locations. The computational system 104 is shown as being in communication with an optional hardware interface 106 and an optional user interface 108. The hardware interface 106, if present, may be used for communicating or controlling other components such as a medical imaging system. The optional user interface 108 may enable a user or operator to control the operation and function of the medical system 100. The computational system 104 is shown as being in communication with a persistent storage 110 which may also be considered to be a memory. The persistent storage or memory 110 is intended to represent a storage medium that is accessible to the computational system 104.

The memory 110 is shown as storing machine-executable instructions 112. The machine-executable instructions 112 enable the computational system 104 to provide basic computational and numerical processing tasks. In addition, the machine-executable instructions 112 may also enable the computational system 104 to control other components via the hardware interface 106. The persistent storage or memory 110 is further shown as containing medical image data 114. The medical image data could for example have been acquired by a medical imaging system controlled by the medical system 100 or it may have been received via a storage medium or a network connection or interface. The persistent storage or memory 110 is further shown as containing an initialized neural network 116. The persistent storage or memory 110 is further shown as containing a trained neural network 118. The trained neural network 118 was provided by training the initialized neural network 116 with the medical image data 114. The trained neural network 118 is able to reproduce the medical image data. The trained neural network 118 is configured to receive a set of continuously variable coordinates and in response, to output an image value. By repeatedly querying the trained neural network 118 an image can be constructed from the trained neural network 118.

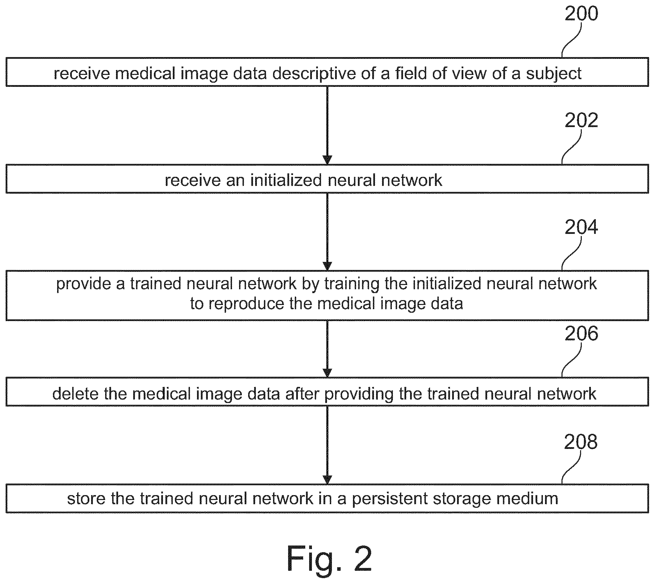

Fig. 2 shows a flowchart which illustrates a method of operating the medical system 100 of Fig. 1. First, in step 200, the medical image data 114, which is descriptive of a field of view of the subject, is received. Next, in step 202, the initialized neural network 116 is received. This may for example involve creating an instance of the initialized neural network 116. In some examples, the initialized neural network 116 may be created by putting random values into the set of weights for the initialized neural network 116. In other examples, an already trained neural network which has an image which resembles or is similar to the medical image data 114 may be chosen. This may for example reduce the amount of training time in some examples.

Next, in step 204, a trained neural network 118 is provided by training the initialized neural network 116 to reproduce the medical image data. For example, a voxel or pixel of the medical image data 114 can be selected. This is used as a ground truth data. The coordinate of this pixel or voxel is then input into the initialized neural network 116 and the difference between the outputted image value and the actual value of the original pixel or voxel is compared and used to train this. This process may be repeated over and over until the initialized neural network accurately reproduces the medical image data, in which case it is then the trained neural network. Next, in step 206, the medical image data 114 is optionally deleted after providing the trained neural network. After this step is performed the memory in the persistent storage medium may be conserved because the trained neural network 118 may consume less storage space than the original medical image data. Finally, in step 208, the trained neural network 118 is stored in the persistent storage medium 110.

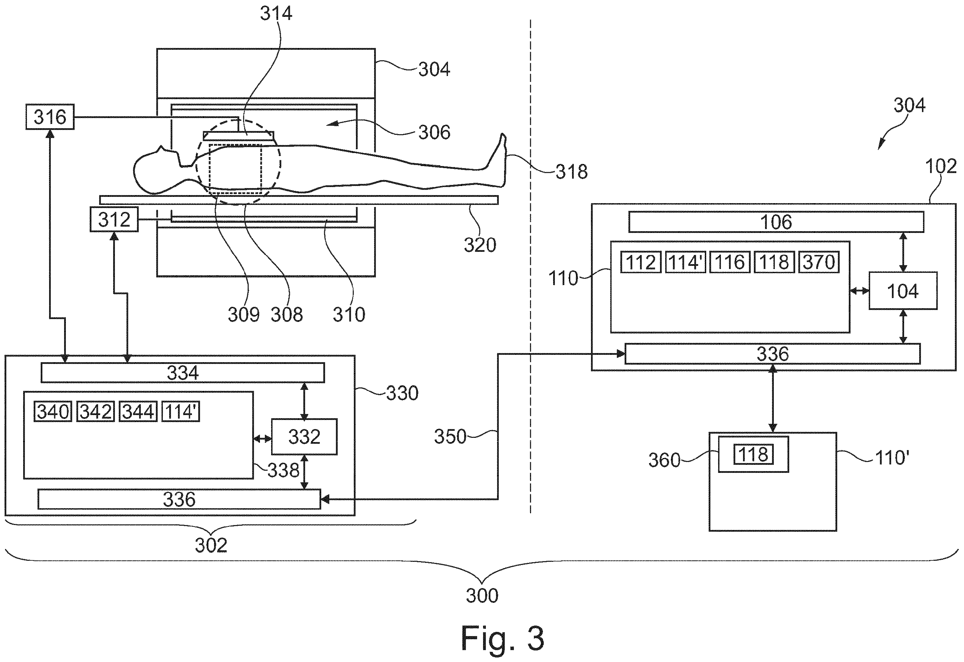

Fig. 3 illustrates a further example of a medical system 300. The example illustrated in Fig. 3 is similar to the example illustrated in Fig. 1 except that it additionally comprises a magnetic resonance imaging system 302.

The magnetic resonance imaging system 302 comprises a magnet 304. The magnet 304 is a superconducting cylindrical type magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data are acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for the acquisition of measured k-space data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

The magnetic resonance imaging system 302 is shown as being controlled by a local controller 330 which may for example be a computer system. The local controller 330 has a local computational system 332 that is in communication with a local hardware interface 334 and a local network interface 336. The local computational system 332 is shown as being in further communication with a local memory 338.

The local memory 338 is shown as storing local machine-executable instructions 340 which enable the local computational system 332 to perform such tasks as controlling the magnetic resonance imaging system 302 via the hardware interface 334 and provide basic numerical and image processing tasks. The local memory 338 is further shown as containing pulse sequence commands 342 which are an example of control commands for controlling a medical imaging system. The local memory 338 is further shown as containing k-space data 344 that was acquired by controlling the magnetic resonance imaging system 302 with the pulse sequence commands 342. The k-space data 344 is an example of measurement data. The memory 338 is further shown as containing a magnetic resonance image 114'. The magnetic resonance image 114' is an example of medical image data.

In this example, the computer 102 comprises a network interface 336 which is used to perform a network connection 350 with the network interface 336 of the local controller 330. The computer 102 in this example could be a cloud-based system. In this example the magnetic resonance image 114' was used to train the trained neural network 118. The network interface 336 of the computer 102 is further shown as being in communication with a persistent storage medium 110'. The persistent storage medium 110' stores a data structure 360 which is shown as encapsulating or storing the trained neural network 118. The trained neural network 118 effectively functions as a data compression for the original magnetic resonance image 114'. The memory 110 is further shown as containing a medical image 370 that has been reconstructed from the trained neural network 118 contained in the data structure 360.

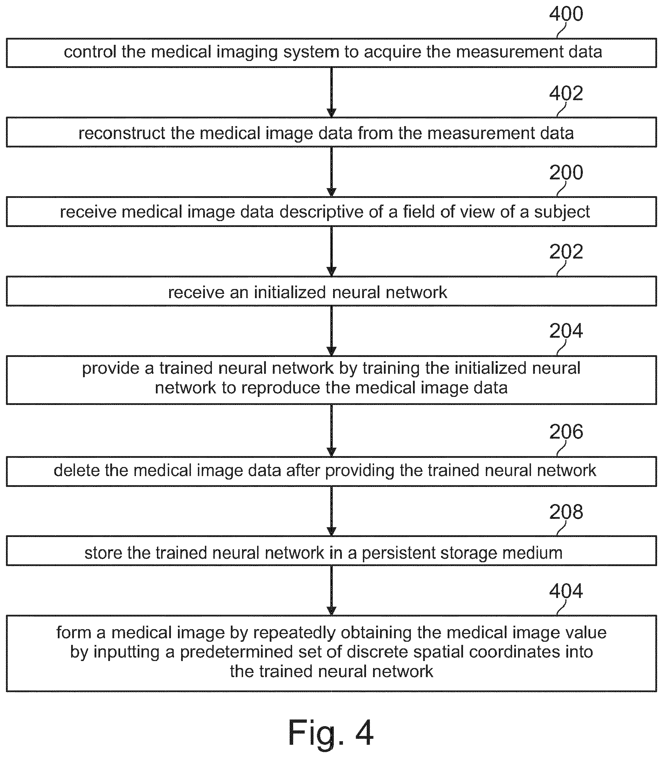

Fig. 4 shows a flowchart which illustrates a method of operating the medical system 300 of Fig. 3. First, in step 400, the medical imaging system, in this case a magnetic resonance imaging system 302 is controlled to acquire the measurement data 344. In this case the measurement data is k-space data 344. Next, in step 402, the medical image data, which is in this case a magnetic resonance image 346, is reconstructed from the k-space data 344. After step 402, steps 200, 202, 204, 206, and 208 are performed as was illustrated in Fig. 2. After step 208 is performed step 404 is performed. In step 404, the medical image 370 is formed by repeatedly obtaining the medical image value by inputting a predetermined set of discrete spatial coordinates into the trained neural network 118.

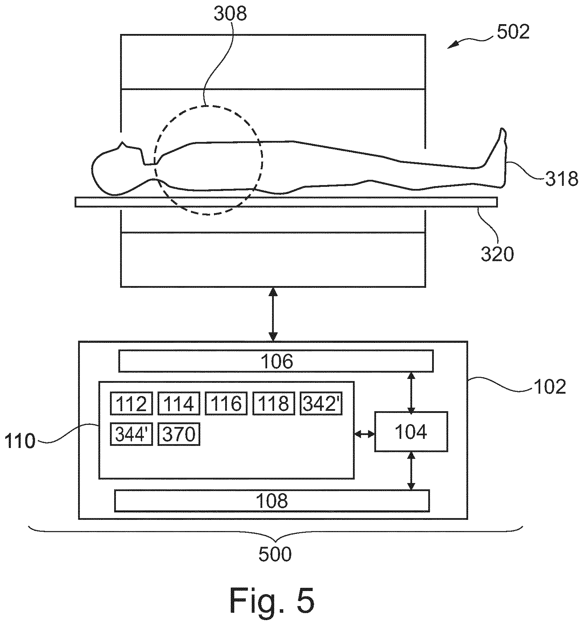

Fig 5 illustrates a further example of the medical system 500. The medical system 500 is similar to the medical system 300 illustrated in Fig. 3 except that the functionality of the local controller 330 and the computer system 102 have been combined into a single computer system 102. Additionally, the magnetic resonance imaging system 302 has been replaced with a medical imaging system 502. The medical imaging system 502 is intended to represent a variety of different types of medical imaging systems. The medical imaging system 502 may be, but is not limited to: a magnetic resonance imaging system, a position emission tomography system, a single photon emission tomography system, an x-ray system, a fluoroscope system, a computed tomography system, an ultrasound imaging system, an intravascular ultrasound system, and an optical coherence tomography system.

The methods illustrated in Figs. 2 and 4 may be performed using the medical system 500.

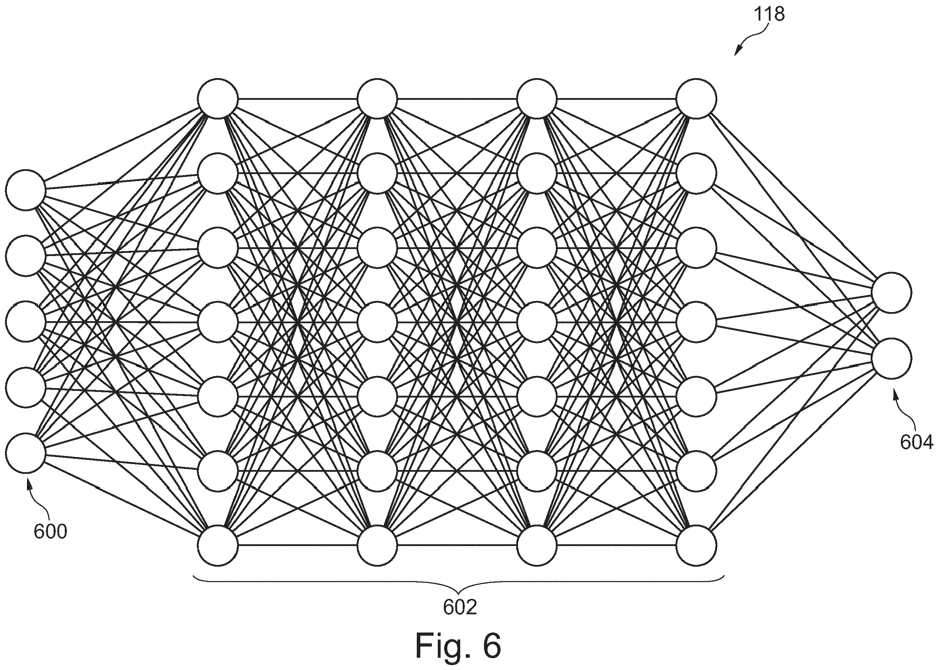

Fig. 6 illustrates an architecture of a neural network that may be used to implement the trained neural network 118. The trained neural network 118 is shown as comprising an input layer 600 that comprises a number of input nodes. The input layer then feeds into multiple fully connected layers 602. The last fully connected layer is then connected to an output layer 604. The input layer 600 comprises an input node for each coordinate. There may for example be two or three nodes corresponding to two or three-dimensional data, there may be an additional node for a time coordinate and there may also be an additional node to use as a selector between different sets of image data. In this example the input layer 600 is connected directly to the fully connected layers 602.

There may however be additional layers to condition the input 600. The storage size of the trained neural network 118 is essentially determined by the number of fully connected layers 602 and the number of nodes in each of these fully connected layers 602. When the initialized neural network is created its memory storage size can be determined by selecting the number of fully connected layers 602 and the number of nodes in each of these layers. The fully connected layers 602 are shown as being connected directly to the output layer 604. The number of nodes will depend upon the type of data that is output. If there is a single value or a contrast output there will be a single node. In this example there are two nodes which should be useful for encoding complex data. In addition, there may also be additional layers between the fully connected layers 602 and the output layer 604 such as softmax or other layers used to condition or format the output layer 604.

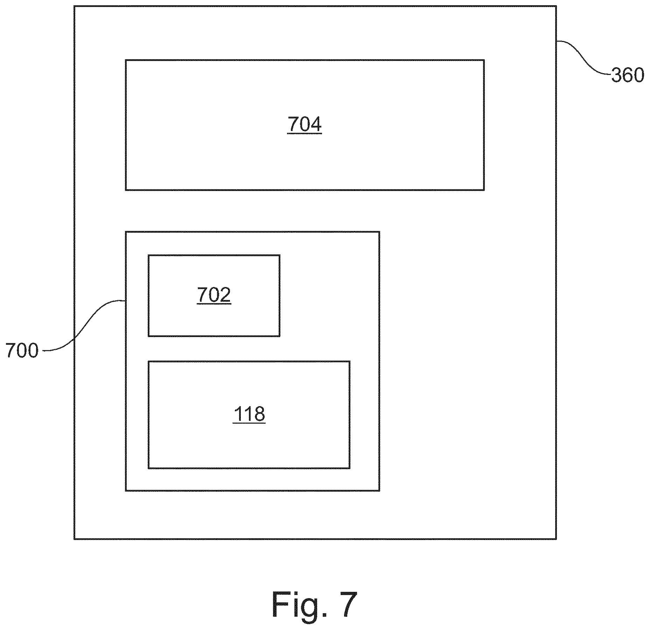

Fig. 7 illustrates an example of a data structure 360. The data structure may comprise an image record 700 that comprises an image identifier 702 and the trained neural network 118. The image identifier 702 may contain data which identifies or describes the medical image data that was encoded in the trained neural network 118. In some instances, the image identifier 702 may also contain metadata which is descriptive of the architecture of the trained neural network 118. The trained neural network 118 may in some instances only specify the weights if a standard neural network architecture is used. In other examples this needs to be specified. The data structure 360 is also shown as containing additional data 704, for example this may contain comments from physicians, additional image data or even additional trained neural networks. In some examples the data structure 360 is a DICOM file.

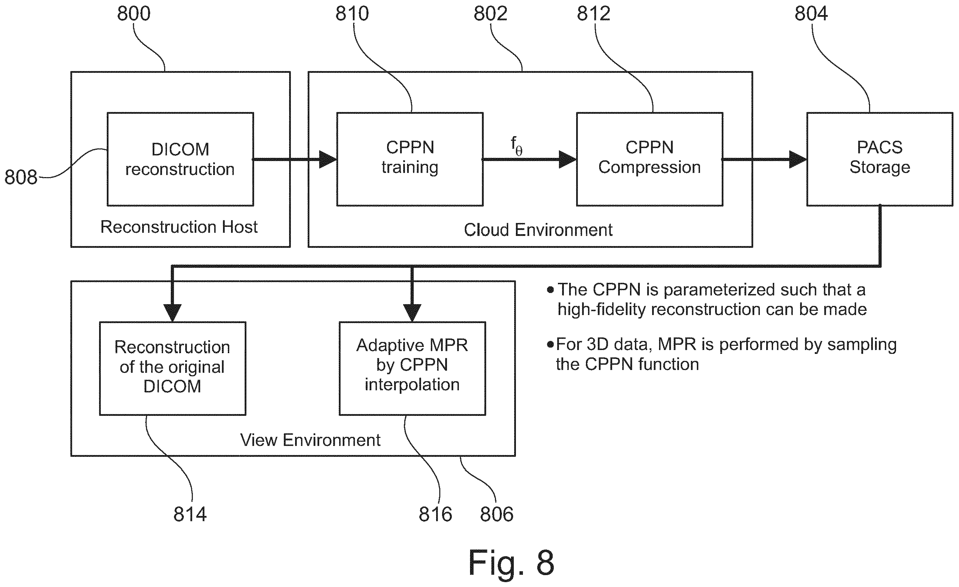

Fig 8 shows a flowchart which illustrates a compression scheme using the trained neural network. The flowchart shows a reconstruction host which feeds into a cloud environment 802 which then stores data in a PACS storage 804. From the PACS storage 804 data can be fed to a new environment 806.

The reconstruction host 800 comprises a module for performing DICOM reconstruction 808. The medical image data from the DICOM reconstruction 808 is fed to a CPPN training 810 which trains the trained neural network 118. The CPPN training 810 feeds into a CPPN compression 812 which is then stored in the PACS storage 804. Within the view environment 806 the trained neural network 118 can be used for reconstruction of the original DICOM image 814 or it may also be used for multi-planar reformation of the image on demand 816. A DICOM image which is created on the reconstruction host. The technician at the scanner acknowledges the quality of the image and decide to proceed in archiving it in the PACS for later use by the radiologist.

Before the image is archived in the PACS (804), it is moved into a cloud environment, where the image is transformed into a parameterized representation of the image. This is performed using a neural network which is optimized to overfit on the data at hand. More specifically, the function $f_\theta$ parameterized by the learnable parameters $\theta$, is trained such:

$$f_\theta(x, y, z) = I(x, y, z)$$

Where $x, y, z$ are coordinates in the image $I$, and $I(x, y, z)$ is the corresponding image intensity. The function, which is also known a Compositional Pattern Producing Network (CPPN), is optimized in the cloud environment and it is implemented, e.g., using a multi-layer perceptron architecture (MLP). Optionally, the MLP can be further compressed using several optimization approaches, such as sparse MLP optimization or network weight quantization.

After the CPPN is generated, it is stored in the PACS instead of the original DICOM. Depending on the desired output quality level, the CPPN take a fraction of the original image disk space (1/3 is used in the experiments presented below).

For viewing the image, the CPPN is used to redraw the image. This is simply computed by executing the function n $f_\theta(x, y, z)$ on the desired range of coordinates.

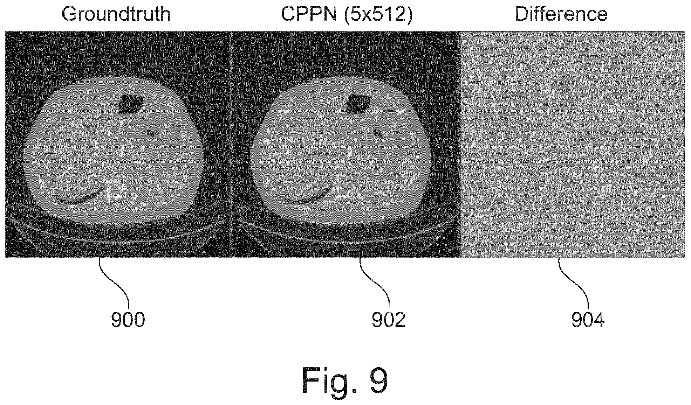

Fig. 9 shows the results of image compression using a relatively small trained neural network 118. The image 900 is the ground truth image. Image 902 shows an image that was reconstructed from a trained neural network 118 that had five connected layers with 512 nodes each. The difference between image 900 and 902 is shown in image 904. It can be seen that this relatively small trained neural network 118 does an excellent job of reproducing the original image 900.

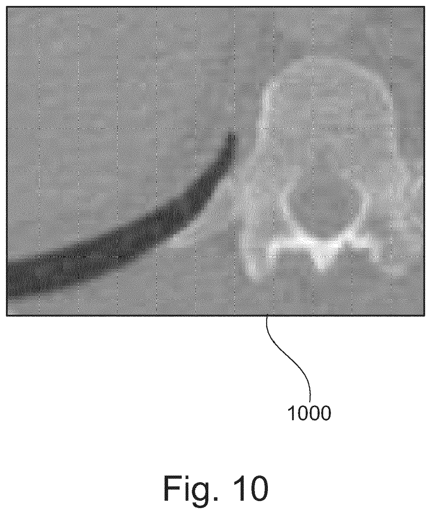

Fig. 10 shows an image 1000 that has been compressed using strong or extreme jpeg compression. Examining this image it can be seen that there are very strong lock artifacts which are visible.

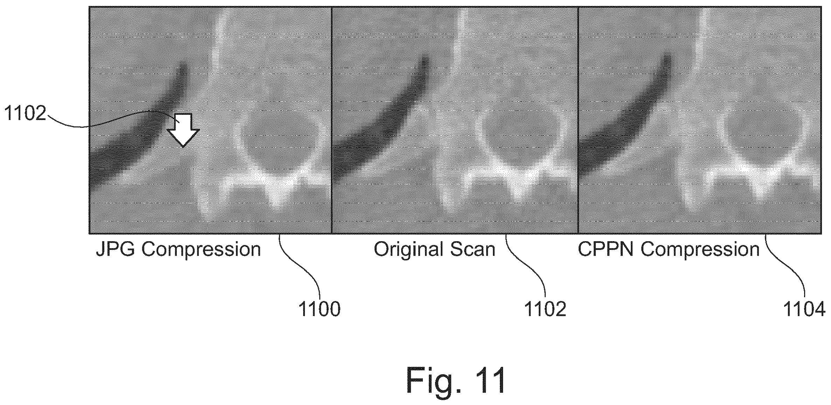

Fig. 11 compares three images. Image 1102 is the original medical image. Image 1100 is a jpeg compression. The block-like artifacts in the jpeg image 1100 become apparent in some anatomical situations. The arrow 1101 marks an area where the jpeg compression causes an image artifact. The image 1104 shows the same data being compressed using a trained neural network 118. It can be seen in image 1104 that the artifact 1102 present in image 1100 is not present.

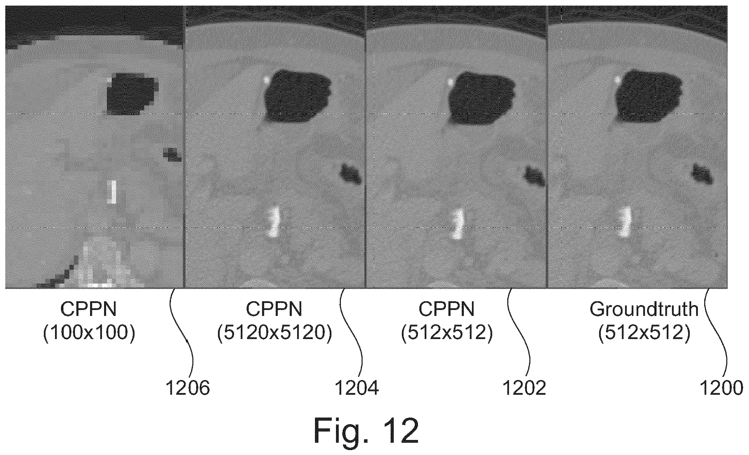

Fig. 12 illustrates how the trained neural network 118 can be used for image interpolation to choose an arbitrary resolution. There are four images which are shown. Image 1200 is the ground truth image; it is the original medical image and has a resolution of 512 x 512. Image 1202 is a first image reproduced using a trained neural network. It has the same resolution as the original and comparing it you can see that it approximates it very well. Image 1204 is a super resolution version that was produced using the trained neural network 118. It has a resolution of 5120 x 5120. Comparing image 1204 to image 1200 is can be seen that this super resolution interpolation did not add any artifacts. Image 1206 shows a lower resolution version. Image 1206 is an image with a resolution of 100 x 100 that was produced using the trained neural network 118. This lower resolution obviously has less detail but it does not display any added artifacts.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

REFERENCE SIGNS LIST

| | |
|---|---|
| 100 | medical system |
| 102 | computer |
| 104 | computational system |
| 106 | hardware interface |
| 108 | user interface |
| 110 | persistent storage / memory |
| 110' | persistent storage |
| 112 | machine executable instructions |
| 114 | medical image data |
| 114' | magnetic resonance image |
| 116 | initialized neural network |
| 118 | trained neural network |
| 200 | receive medical image data descriptive of a field of view of a subject |
| 202 | receive an initialized neural network |
| 204 | provide a trained neural network by training the initialized neural network to reproduce the medical image data |
| 206 | delete the medical image data after providing the trained neural network |
| 208 | store the trained neural network in a persistent storage medium |
| 302 | magnetic resonance imaging system |
| 304 | magnet |
| 306 | bore of magnet |
| 308 | imaging zone |
| 309 | field of view |
| 310 | magnetic field gradient coils |
| 312 | magnetic field gradient coil power supply |
| 314 | radio frequency coil |
| 316 | transceiver |

| | |
|---|---|
| 318 | subject |
| 320 | subject support |
| 330 | local controller |
| 332 | local computational system |
| 334 | local hardware interface |
| 336 | network interface |
| 338 | local memory |
| 340 | local machine executable instructions |
| 342 | pulse sequence commands |
| 342' | control commands |
| 344 | k-space data |
| 344' | measurement data |
| 346 | magnetic resonance image |
| 350 | network connection |
| 360 | data structure |
| 370 | medical image |
| 400 | control the medical imaging system to acquire the measurement data |
| 402 | reconstruct the medical image data from the measurement data |
| 404 | form a medical image by repeatedly obtaining the medical image value by inputting a predetermined set of discrete spatial coordinates into the trained neural network |
| 500 | medical system |
| 502 | medical imaging system |
| 600 | input layer |
| 602 | multiple fully connected layers |
| 604 | output layer |
| 700 | image record |
| 702 | image identifier |
| 704 | additional data |
| 800 | reconstruction host |
| 802 | cloud environment |
| 804 | PACS storage |
| 806 | view environment |
| 808 | DICOM reconstruction |
| 810 | CPPN (neural network) training |
| 812 | CPPN (neural network) compression |
| 814 | reconstruction of the origin DICOM |
| 816 | Adaptive MPR by CPPN interpolation |
| 900 | ground truth image |
| 902 | reconstructed image |
| 904 | difference |
| 1000 | jpeg compressed image |
| 1100 | JPEG compressed image |
| 1102 | original image |
| 1104 | neural network compressed image |
| 1200 | ground truth or original image with resolution of 512x512 |
| 1202 | neural network reconstructed image with resolution of 512x512 |
| 1204 | neural network reconstructed image with resolution of 5120x5120 |
| 1206 | neural network reconstructed image with resolution of 100x100 |

**Claims**

1. A medical system (100, 300, 500) comprising:

   - a memory (110) storing machine executable instructions (112),
   - a computational system (104), wherein execution of the machine executable instructions causes the computational system to:

      - receive (200) medical image data (114) descriptive of a field of view (309) of a subject (318);
      - receive (202) an initialized neural network (116), wherein the initialized neural network is configured to output an image value in response to receiving a set of continuously variable coordinates spanning the field of view of the subject;
      - provide (204) a trained neural network (118) by training the initialized neural network to reproduce the medical image data; and
      - store (208) the trained neural network in a persistent storage medium (110, 110', 804).

2. The medical system of claim 1, wherein execution of the machine executable instructions further causes the computational system to delete (206) the medical image data after providing the trained neural network.

3. The medical system of any one of the preceding claims, wherein the medical image data comprises multiple image types of the field of view, wherein the set of continuously variable coordinates comprises an image selection coordinate used to select one of the multiple image types.

4. The medical system of any one of the preceding claims, wherein the trained neural network comprises multiple fully connected layers (602), and wherein the trained neural network preferably comprises 3, 4, 5, or 6 fully connected layers.

5. The medical system of claim 4, wherein the multiple fully connected layers each have fewer than 10000 nodes, and wherein the multiple fully connected layers preferably each have below 5000, 3000, 2000, or 1000 nodes.

6. The medical system of any one of the preceding claims, wherein the initialized neural network is trained with the medical image data using either a back propagation training algorithm or a genetic algorithm optimization technique and/or wherein the trained neural network has a memory storage size less than a memory storage size of the medical image data.

7. The medical system of any one of the preceding claims, wherein trained neural network is stored in the persistent storage medium by placing it into a data structure (360), and wherein the data structure is preferably a Digital Imaging and Communications in Medicine file, and wherein the medical image data and the image value are preferably complex valued.

8. The medical system of any one of the preceding claims, wherein the medical system further comprises a medical imaging system (302, 502) configured to acquire measurement data (344, 344') descriptive of the field of view of the subject, wherein execution of the machine executable instructions further causes the computational system to:

   - control (400) the medical imaging system to acquire the measurement data; and
   - reconstruct (402) the medical image data from the measurement data.

9. The medical system of claim 8, wherein the medical imaging system is any one of the following: a magnetic resonance imaging system (302), a position emission tomography system, a single photon emission tomography system, an x-ray system, a fluoroscope system, a computed tomography system, an ultrasound imaging system, an intravascular ultrasound system, and an optical coherence tomography system.

10. The medical system of any one of the preceding claims, wherein execution of the machine executable instructions further causes the computational system to form (404) a medical image by repeatedly obtaining the medical image value by inputting a predetermined set of discrete spatial coordinates into the trained neural network, wherein the predetermined set of discrete spatial coordinates spans at least a portion of the medical image; and wherein the predetermined set of discrete spatial coordinates preferably has a spatial resolution higher than the medical image data.

11. The medical system of claim 10, wherein the medical image data comprises three-dimensional image data, and wherein the predetermined set of discrete coordinates forms a two-dimensional surface.

12. The medical system of any one of the preceding claims, wherein the medical image data is time dependent, wherein the set of continuously variable coordinates comprises a time coordinate.

13. A data structure (360) for storing at least one image, wherein the data structure comprises an image identifier (702) and a set of weights for a trained neural network, wherein the trained neural network is configured to output an image value in response to receiving a set of continuously variable coordinates spanning the at least one image, and wherein the image is readable by repeatedly inputting the set of continuously variable coordinates.

**14.** A medical imaging method, wherein the method comprises:

- receiving (200) medical image data (114) descriptive of a field (309) of view of a subject;
- receiving (202) an initialized neural network (116), wherein the initialized neural network is configured to output an image value in response to receiving a set of continuously variable coordinates spanning the field of view of the subject;
- providing (204) a trained neural network (118) by training the initialized neural network to reproduce the medical image data; and
- storing (208) the trained neural network in a persistent storage medium (110, 110', 804).

**15.** A computer program comprising machine executable instructions (112) for execution by a computational system (104), wherein execution of the machine executable instructions causes the computational system to:

- receive (200) medical image data (114) descriptive of a field of view (309) of a subject (318);
- receive (202) an initialized neural network (116), wherein the initialized neural network is configured to output an image value in response to receiving a set of continuously variable coordinates spanning the field of view of the subject;
- provide (204) a trained neural network (118) by training the initialized neural network to reproduce the medical image data; and
- store (208) the trained neural network in a persistent storage medium (110, 110', 804).

Fig. 1

200

receive medical image data descriptive of a field of view of a subject

202

receive an initialized neural network

204

provide a trained neural network by training the initialized neural network to reproduce the medical image data

206

delete the medical image data after providing the trained neural network

208

store the trained neural network in a persistent storage medium

Fig. 2

Fig. 3

EP 4 345 834 A1

400

| control the medical imaging system to acquire the measurement data |

402

| reconstruct the medical image data from the measurement data |

200

| receive medical image data descriptive of a field of view of a subject |

202

| receive an initialized neural network |

204

| provide a trained neural network by training the initialized neural network to reproduce the medical image data |

206

| delete the medical image data after providing the trained neural network |

208

| store the trained neural network in a persistent storage medium |

404

| form a medical image by repeatedly obtaining the medical image value by inputting a predetermined set of discrete spatial coordinates into the trained neural network |

## Fig. 4

Fig. 5

Fig. 6

Fig. 7

Fig. 8

The CPPN is parameterized such that a high-fidelity reconstruction can be made

For 3D data, MPR is performed by sampling the CPPN function

EP 4 345 834 A1

Groundtruth 900    CPPN (5x512) 902    Difference 904

Fig. 9

1000

Fig. 10

Fig. 11

Fig. 12

Europäisches
Patentamt

European
Patent Office

Office européen
des brevets

# EUROPEAN SEARCH REPORT

**Application Number**

EP 22 19 8722

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EMILIEN DUPONT ET AL: "COIN++: Neural Compression Across Modalities", ARXIV.ORG, CORNELL UNIVERSITY LIBRARY, 201 OLIN LIBRARY CORNELL UNIVERSITY ITHACA, NY 14853, 10 June 2022 (2022-06-10), XP091244739, | 1-10, 13-15 | INV. G16H30/20 G16H50/20 |
| Y | * p. 1, title p. 1, Abstract, lines 4-6 p. 1, Fig. 1 and description thereof p. 2 lines 1f p. 2, second paragraph, lines 1f p. 3, first paragraph, lines 1f p. 3, Figure 3 and description thereof p. 5, first paragraph, lines 3f p. 5, first paragraph, lines 7f p. 5, description of Fig. 4 p. 6, section "Implicit neural representations and compressions" p. 10, section "Medical data brain MRI scans", lines 1f p. 11, Fig. 11 p. 26, Fig. 18 * | 11,12 | |
| A | QING WU ET AL: "An Arbitrary Scale Super-Resolution Approach for 3-Dimensional Magnetic Resonance Image using Implicit Neural Representation", ARXIV.ORG, CORNELL UNIVERSITY LIBRARY, 201 OLIN LIBRARY CORNELL UNIVERSITY ITHACA, NY 14853, 27 October 2021 (2021-10-27), XP091082057, * Abstract, lines 16 - 22 * | 10 | |

-----

-----

-/--

TECHNICAL FIELDS
SEARCHED (IPC)

G16H

The present search report has been drawn up for all claims

| Place of search | Date of completion of the search | Examiner |
|---|---|---|
| Munich | 10 March 2023 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 19 8722

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PAK-HEI YEUNG ET AL: "ImplicitVol: Sensorless 3D Ultrasound Reconstruction with Deep Implicit Representation", ARXIV.ORG, CORNELL UNIVERSITY LIBRARY, 201 OLIN LIBRARY CORNELL UNIVERSITY ITHACA, NY 14853, 24 September 2021 (2021-09-24), XP091059700, * p. 3, lines 5-13 * | 11,12 | |
| A | EMILIEN DUPONT ET AL: "COIN: COmpression with Implicit Neural representations", ARXIV.ORG, CORNELL UNIVERSITY LIBRARY, 201 OLIN LIBRARY CORNELL UNIVERSITY ITHACA, NY 14853, 10 April 2021 (2021-04-10), XP081929782, * the whole document * | 1-15 | |
| A | CHABRIAIS J ET AL: "DICOM, the standard for medical imaging", EMC - RADIOLOGIE, ELSEVIER, vol. 1, no. 6, 28 October 2004 (2004-10-28), pages 577-603, XP004656161, ISSN: 1762-4185, DOI: 10.1016/J.EMCRAD.2004.09.002 * the whole document * | 7 | TECHNICAL FIELDS SEARCHED (IPC) |
| A | ELIZABETH FONS ET AL: "HyperTime: Implicit Neural Representation for Time Series", ARXIV.ORG, CORNELL UNIVERSITY LIBRARY, 201 OLIN LIBRARY CORNELL UNIVERSITY ITHACA, NY 14853, 11 August 2022 (2022-08-11), XP091292619, * the whole document * | 12 | |

The present search report has been drawn up for all claims

| Place of search | Date of completion of the search | Examiner |
|---|---|---|
| Munich | 10 March 2023 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MUELLER.** Instant Neural Graphics Primitives with a Multiresolution Hash Encoding. *arXiv:2201.05989*

- *ACM Trans. Graph,* 2022, vol. 41